# EUROPEAN PATENT APPLICATION

(11) **EP 1 767 648 A1**
(43) Date of publication of application: **28.03.2007**
(21) Application number: 05743655.2
(22) Date of filing: 27.05.2005
(51) Int. Cl.: C12Q 1/48, C12M 1/34

(54) **METHOD FOR MEASURING ENZYME ACTIVITY AND COLUMN FOR USE IN MEASURING ENZYME ACTIVITY**

(30) Priority: 01.06.2004 JP 2004163367
(71) Applicant: SYSMEX CORPORATION, Kobe-shi, Hyogo 651-0073 (JP)
(72) Inventor: Nakayama, Satoshi, Sysmex Corporation, Kobe-shi, Hyogo 6510073 (JP); Nagaoka, Kanako, Sysmex Corporation, Kobe-shi, Hyogo 6510073 (JP); Kobayashi, Hironori, Sysmex Corporation, Kobe-shi,Hyogo 6510073 (JP); Ishihara, Hideki, Sysmex Corporation, Kobe-shi, Hyogo 6510073 (JP)
(74) Representative: De Clercq, Ann G. Y.
(86) International application number: PCT/JP2005/009789
(87) International publication number: WO 2005/118842

(57) **Abstract**

A method of measuring an enzyme activity, which comprises the steps of:
(1) introducing a sample containing an enzyme to be measured into a column packed with a packing material comprising a water-insoluble support and an antibody which is immobilized onto the support and which recognizes the enzyme to be measured , to thereby capture the enzyme with the antibody;
(2) introducing a substrate for the enzyme into the column to react the enzyme captured by the antibody with the substrate; and
(3) detecting a product obtained by the reaction.

The above method allows the rapid measurement of an enzyme activity with good reaction efficiency.

## Description

### TECHNICAL FIELD

The present invention relates to a method for measuring an enzyme activity, especially cyclin-dependent kinases, and a column for measuring an enzyme activity.

### BACKGROUND ART

Enzyme activity is conventionally measured by analyzing the amount of a substrate decreased or the amount of a reaction product increased due to a reaction between the enzyme and substrate. In order to measure the activity of a specific enzyme in a sample, such as a biological sample, which also contains various impurities, such as inhibitory enzymes, other than the enzyme to be measured, it is necessary to carry out a step for purifying the target enzyme to be measured in order to eliminate the effect of impurities such as enzymes inhibiting a target enzyme reaction. Such step is carried out using, for example, immunoprecipitation technique by mixing a sample containing a target enzyme to be measured and a water-insoluble support to which an antibody specific to the enzyme has been immobilized, to thereby isolate the target enzyme from the sample.

As a method for measuring an enzyme activity by carrying out enzyme reaction after the immunoprecipitation such as mentioned above, there is disclosed a method for measuring the activity of phosphoenzymes referred to as cyclin-dependent kinases (CDKs) which are cell cycle regulatory factors for controlling cell growth (U.S. Patent Publication No. 2002/0164673).

CDKs usually exist in cytoplasm of a cell as inactive members, and CDKs themselves are activated by phosphorylation, for example, and move into a nucleus of a cell. In the nucleus, the CDKs bind to cyclin molecules existing in the nuclei to form complexes of CDK and cyclin (hereinafter, referred to as "activated CDKs") and these complexes positively regulate the progress of the cell cycle at various stages of the cell cycle. As described above, the CDKs and cyclins are closely related to each other, and it is also known that the promotion of expression of cyclin genes correlates with the progress or malignancy of various cancers (Wataru Yasui, Sysmex Journal International, Vol. 10, No. 2, pp. 85-92, 2000). Therefore, it is expected that measuring the activities of the various types of CDKs will provide good indices of the type and malignancy of diseases related to the control of the cell cycle.

The method of measuring CDK activity of the above U.S. Patent Publication No. 2002/0164673 is the one carried out by mixing a biological sample, an anti-CDK antibody and protein A beads to cause an immunoprecipitation, followed by reacting a substrate protein which is a substrate for CDK and adenosine 5'-O-(3-thiotriphosphate) (ATP-γS) in the presence of CDK to introduce a monothiophosphate group into the substrate protein, allowing a labeling fluorescent substance or a labeling enzyme to bind to a sulfur atom of the introduced monothiophosphate group to label the substrate protein, and measuring the amount of the labeled substrate protein, to thereby measure CDK activity.

As the other methods for measuring an enzyme activity, there is known a method for measuring an enzyme activity by introducing a nicotinamide adenine dinucleotide (NAD)-requiring enzyme and a substrate for the enzyme into a column packed with a packing material for measuring the enzyme activity obtained by immobilizing NAD onto a water-insoluble support and determining the amount of an obtained product (Japanese Patent Publication No. Hei 5(1993)-328996). Further, there is known a method for measuring an enzyme activity by introducing a sample into a column packed with a packing material, onto which a substrate has been immobilized to decompose the substrate by reaction of the substrate with the enzyme, and determining the amount of a substrate decomposed product contained in an eluate from the column (U.S. Patent No. 5654152). Furthermore, there is known a method for measuring an enzyme activity by introducing an enzyme to be measured into a column for measuring the enzyme activity packed with a support onto which a substrate has been immobilized via a biotin-avidin bond and determining the amount of an obtained substrate decomposed product (Japanese Patent No. 2810138).

### DISCLOSURE OF THE INVENTION

### PROBLEM TO BE SOLOVED BY THE INVENTION

However, such conventional methods require long time to measure CDK activity, and therefore it is difficult to examine many samples. Therefore, the need was felt for a method of measuring an enzyme activity by which CDK activity can be measured easily in a shorter time.

### MEANS FOR SOLVING THE PROBLEM

The present invention is directed to a method of measuring an enzyme activity, which comprises the steps of:
(1) introducing a sample containing an enzyme to be measured into a column packed with a packing material comprising a water-insoluble support and an antibody which is immobilized onto the support and which recognizes the enzyme to be measured, to thereby capture the enzyme with the antibody;
(2) introducing a substrate for the enzyme into the column to react the enzyme captured by the antibody with the substrate; and
(3) detecting a product obtained by the reaction.

The present invention is also directed to a method of measuring an enzyme activity, which comprises the steps of:
(1) introducing an antibody which recognizes an enzyme to be measured into a column packed with a water-insoluble support, to thereby immobilize the antibody onto the support;
(2) introducing a sample containing the enzyme to be measured into the column, to thereby capture the enzyme with the antibody;
(3) introducing a substrate for the enzyme into the column to react the enzyme captured by the antibody with the substrate; and
(4) detecting a product obtained by the reaction.
   The present invention is also directed to a column for measuring an enzyme activity packed with a packing material comprising a water-insoluble support and an antibody which is immobilized on the support and which recognizes a phosphoenzyme.

### EFFECT OF THE INVENTION

According to the method of measuring an enzyme activity of the present invention, the packing material comprising the support and the antibody is packed into a column and the antibody is allowed to capture an enzyme to be measured, and therefore even when the amount of the target enzyme contained in a sample is small, the enzyme can be captured rapidly. Further, since a substrate is fed into the column, an enzyme reaction can be carried out easily and efficiently.
Furthermore, by measuring CDK activity using the method of measuring an enzyme activity of the present invention, it is possible to significantly reduce the reaction time as compared to a conventional immunoprecipitation method, thus it is possible to carry out a rapid examination.

### BRIEF DESCRIPTION OF THE DRAWINGS

Fig. 1 shows a preferred embodiment of the column which can be used for the method of measuring an enzyme activity of the present invention.
Fig. 1-1 is an exploded view of the column shown in Fig. 1 separated into a reservoir, column body and base part.
Fig. 2 is a graph which shows a comparison of CDK2 activities measured in Example 1 and in Comparative Example 1.
Fig. 3 is a graph which shows the relation between the mass of protein in the sample and CDK2 activity measured by the method of measuring an enzyme activity using the column.
Fig. 4 is a graph which shows the relation between the mass of protein in the sample and CDK2 activity measured by the conventional method of measuring an enzyme activity using immunoprecipitation.
Fig. 5 shows a result of measuring CDK activity of the sample containing 20 µg of protein by use of the method of measuring an enzyme activity using the column.

### DESCRIPTION OF THE REFERENCE NUMERALS

1 Reservoir
2 Column body
3 Base part
4 Contacting part
5 Contacting part

### BEST MODE FOR CARRYING OUT THE INVENTION

The enzyme to be measured in the method of measuring an enzyme activity of to the present invention includes phosphoenzymes such as cyclin-dependent kinases (CDKs), serine/threonine kinases (e.g., PKB, PKC), and tyrosine kinases (e.g., Src, Abl); and proteases such as MMPs (matrix metalloproteases) and caspases. The method of measuring an enzyme activity of the present invention is particularly suitable for measuring phosphoenzymes. Among phosphoenzymes, CDKs are preferred. CDKs include CDK1, CDK2, CDK4, CDK5, CDK6, and CDK7.
The antibody which recognizes the enzyme may either be a monoclonal antibody or a polyclonal antibody, or it may be obtained by recombinant technology.

The method of measuring an enzyme activity of the present invention is carried out using a column. The shape of the column is appropriately selected to improve the reaction efficiency of enzyme reaction. Thus, the shape (e.g., diameter, length) of the column is preferably selected in view of the collision efficiency between the packing material packed in the column and molecules in a sample to be introduced into the column, as well as workability of the column and applicability to a system in which said column is used. From such viewpoints, the diameter of the column is preferably in the range of 0.3 to 30 mm, more preferably 0.5 to 20 mm, still more preferably 0.5 to 15 mm. The length of the column is preferably in the range of 5 to 100 mm, more preferably 10 to 80 mm, still more preferably 10 to 50 mm.

The material of the column is not particularly limited as long as it is not decomposed under conditions for measuring the activity of the enzyme to be measured, it does not have a problem in terms of strength and protein adsorption, and it does not adversely affect the enzyme reaction. Vinyl chloride (PVC), polypropylene (PP) and the like can be used. Columns made of PVC are preferred in that it has high strength, can be easily processed, and can keep the constant inside temperature by virtue of its low heat conductivity. Columns made of PP are preferred in that it has low protein absorption.

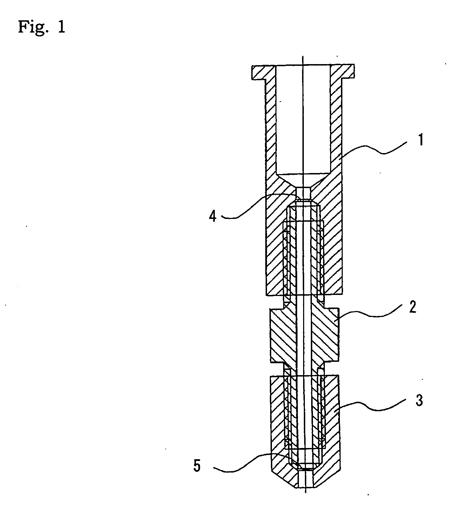

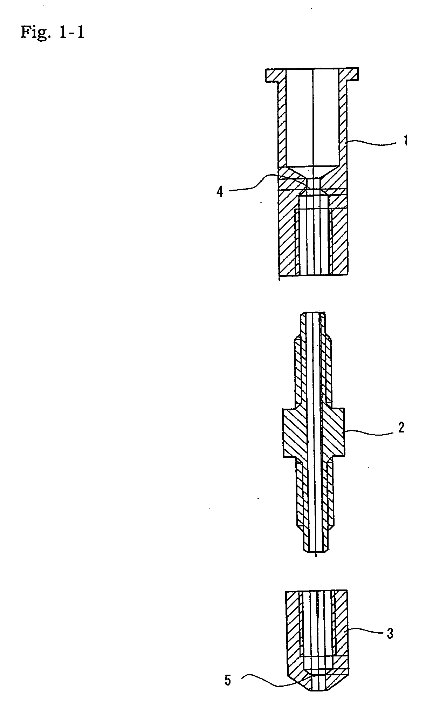

A preferred embodiment of the column that can be used in the present invention is shown in Fig. 1 and Fig. 1-1. As shown in Fig. 1-1, it is preferred that the column has a reservoir 1 for storing a sample at the top of a column body 2, and a base part 3 for preventing leakage of the packing material at the bottom of the column body 2. The column shown in Fig. 1 can be obtained and used by assembling these parts.
A membrane can be used for a contacting part 4 between the reservoir 1 and column body 2 and a contacting part 5 between the column body 2 and base part 3. The material of the membrane is not particularly limited as long as it has low protein absorption and has strength sufficient not to be broken at the time of introducing a liquid into the column. Polyvinylidene fluoride (PVDF), nylon, a glass fiber filter and the like can be used. Among them, PVDF is preferred. As a PVDF membrane, commercially available products, for example, Durapore (manufactured by Millipore Corporation) can be used.

In the method of measuring an enzyme activity of the present invention, the packing material is to be packed in the column body 2. In this specification, the term "packing material" means the one obtained by immobilizing the antibody which recognizes the enzyme to be measured onto the water-insoluble support.

The support to be used in the method of measuring an enzyme activity of the present invention is usually insoluble in water, but is not particularly limited as long as it is not decomposed under conditions for measuring the activity of an enzyme to be measured and it does not adversely affect the enzyme reaction. Polysaccharides such as cellulose, agarose and dextran and silica gel can be used. Among them, agarose is preferably used.
The shape of the support is not particularly limited as long as it can be packed in a column to be used. However, a particle or porous support is preferably used because, as described later, a contact area between an antibody and a sample is increased when the antibody immobilized on the packing material captures the enzyme contained in the sample. The particle diameter is preferably in the range of 0.01 to 0.5 mm, more preferably 0.05 to 0.4 mm, still more preferably of 0.05 to 0.2 mm.
The porous support may be formed so as to fit the shape of the column body, such as a cylindrical shape. The usable porous support may be, for example, monolithic silica.

In the method of measuring an enzyme activity of the present invention, the packing material obtained by immobilizing a substance that can bind to the antibody (hereinafter, referred to as an "antibody-binding substance") on the support and allowing the antibody to bind thereto may be used. The antibody-binding substance includes substances that can recognize part of an antibody, such as Fab fragment, Fc fragment or κ chain, and can bind thereto. Specific examples of such substances include protein A, protein G, protein L, and protein LA. Among them, protein A or protein G is preferred. As protein A and protein G, those obtained from natural products, those produced using gene-recombination technology, or those obtained by modifying thereof can be used. Such modification may be, for example, the one for improving antibody recognition specificity, such as the removal of the albumin-binding region, removal of the cell wall-binding region and the like.

A method for immobilizing the antibody-binding substance on the support includes a method using a reactive compound. As the reactive compound, p-nitrophenylchloroformate, cyanogen bromide, N-hydroxysuccinimide, epoxides and the like can be preferably used. In this case, the reactive compound binds to the support, and then the antibody-binding substance binds to the reactive compound. In this way, the antibody-binding substance can be immobilized onto the support.

As the support onto which the antibody-binding substance has been immobilized, a commercially-available product may also be used. As the support onto which protein A has been immobilized as the substance that can bind to the antibody, Protein A Sepharose FF and Protein A Sepharose HP (manufactured by Amersham) can be used. As the support onto which protein G has been immobilized as the substance that can bind to the antibody, Protein G Sepharose HP (manufactured by Amersham) can be used.

In a preferred embodiment of the method of measuring an enzyme activity of the present invention, first, the packing material comprising the antibody that recognizes the enzyme to be measured and the support is packed in the column. The packing material can be obtained by immersing and incubating the support in a solution obtained by dissolving the antibody in an appropriate buffer. The antibody-binding substance may be previously immobilized onto the support. The temperature for incubation is preferably in the range of 2 to 10°C, more preferably 4 to 6°C. The time for incubation is preferably in the range of 20 to 90 minutes, more preferably 40 to 70 minutes.
Then, the thus obtained packing material may be washed in order to remove the antibody not immobilized on the support. A washing solution is not particularly limited as long as the antibody not immobilized on the support can be removed, and may contain a small amount of albumin or a surfactant. After carrying out such a washing, the packing material can be suspended in an appropriate buffer, and then be packed into a column.

The amount of the antibody to be immobilized on the support can be appropriately selected according to the antibody binding capacity of the support, the type of the enzyme to be measured, etc., and is usually in the range of 0.1 to 100 µg, preferably 0.5 to 50 µg, more preferably 0.7 to 20 µg.

A method for packing the packing material in the column described above is not particularly limited as long as the collision efficiency between the packing material and molecules in the sample introduced into the column is sufficient. For example, a packing material which is particle in shape can be packed in the column by suspending it in an appropriate buffer and packing the material into the column at a constant flow rate. The flow rate for packing the packing material which is particle in shape can be appropriately selected according to the type of the packing material to be used, but is preferably in the range of 10 to 1,000 µl/min, more preferably 20 to 700 µl/min, still more preferably 50 to 500 µl/ min.
The amount of the packing material to be packed in the column is preferably in the range of 10 µl to 15 ml, more preferably 15 µl to 15 ml.

In another preferred embodiment of the method of measuring an enzyme activity of the present invention, the support is packed in the column. After the support is packed in the column at a flow rate similar to the above flow rate at which the packing material is introduced into the column, the antibody which recognizes the enzyme to be measured is introduced into the column. Such an antibody which recognizes the enzyme to be measured can be introduced into the column and immobilized on the support by, for example, dissolving the antibody in an appropriate buffer and feeding the solution into the column at a flow rate such that a predetermined amount of the antibody can be immobilized on the support. The flow rate of the antibody solution is preferably in the range of 5 to 500 µl/min, more preferably 10 to 350 µl/min, still more preferably 25 to 250 µl/min.
The amount of the support to be packed in the column is preferably in the range of 10 µl to 15 ml, more preferably 15 µl to 15 ml.

After the antibody is immobilized on the support, the support may be washed with the washing solution as described above to remove the antibody remaining in the column and not immobilized on the support. The flow rate of the washing solution is preferably in the range of 5 to 500 µl/min, more preferably in the range of 10 to 200 µl/ min.

The thus obtained column for measuring an enzyme activity in which the packing material comprising the water-insoluble support and the antibody which is immobilized onto the support and which recognizes a phosphoenzyme is packed is one aspect of the present invention. This column for measuring an enzyme activity can be stored at 2 to 10°C and can be used for the measurement of an enzyme activity by carrying out the following steps at the time of use thereof.

Into the column obtained through the processes described above, the sample containing the enzyme to be measured is introduced to thereby capture the enzyme with the antibody.
The sample containing the enzyme to be measured may be a purified sample containing only the target enzyme, a crude purified sample or a biological sample. In a case where a biological sample is used, the biological sample may be roughly purified by separation chromatography in advance. In a case where the enzyme to be measured is CDK, the sample can be prepared by, for example, subjecting living cells contained in a biological sample to chemical or physical treatment to break their cell wall and nuclear membrane. Such chemical or physical treatment may preferably be carried out, for example, in a buffer containing a surfactant, protease inhibitor, and dephosphorylating enzyme inhibitor by electric homogenization using a Waring blender, or suction and ejection with a syringe, or ultrasonication.
The flow rate for feeding the sample into the column is preferably in the range of 5 to 500 µl/min, more preferably 10 to 350 µl/min, still more preferably 25 to 250 µl/min.

After the sample is introduced into the column to allow the enzyme to be captured, it is preferable to carry out washing in order to remove the enzyme remaining in the column and not captured by the antibody and other impurities contained in the sample. In a case where the enzyme to be measured is CDK, a washing solution may contain magnesium chloride for improving reactivity of the enzyme with the substrate, which is described herein below, or may contain dithiothreitol (DTT) as an enzyme stabilizer.
The flow rate of the washing solution is preferably in the range of 5 to 500 µl/min, more preferably 10 to 200 µl/min.
It is desirable that washing is carried out for 3 to 30 minutes, preferably 5 to 15 minutes in order to prevent adverse effect of the free enzyme remaining in the column and not captured by the antibody and impurities on enzyme reaction, which is described herein below.

The substrate for the enzyme is introduced into the column in which the enzyme has been captured to carry out the enzyme reaction. The substrate is not particularly limited as long as a reaction product can be produced as a result of contact between the substrate and enzyme to be measured. The substrate may be one or two or more species. In a case where two or more species of substrates are used, they are fed into the column simultaneously or separately.
The enzyme reaction can be carried out by introducing the substrate solution obtained by dissolving the substrate in an appropriate buffer into the column. The flow rate of the substrate solution can be appropriately selected according to the kind of enzyme to be measured, the kind of substrate to be used, the size of a column to be used, etc., but is usually in the range of 0. to 200 µl/min, preferably 0.5 to 150 µl/min, more preferably 1 to 100 µl/min.
The pH of the substrate solution and the temperature for enzyme reaction can be selected according to the kind of enzyme to be measured and the kind of substrate to be used so that the reaction conditions of the enzyme to be measured are optimized. For example, in a case where the enzyme to be measured is CDK, the pH is preferably in the range of 6.5 to 8.5, more preferably 7.0 to 7.4, and the reaction temperature is preferably in the range of 25 to 40°C, more preferably 37°C.

In a case where the enzyme to be measured is CDK, substrate protein and adenosine 5'-O-(3-thiotriphosphate) (ATP-γS) are preferably used as substrates.

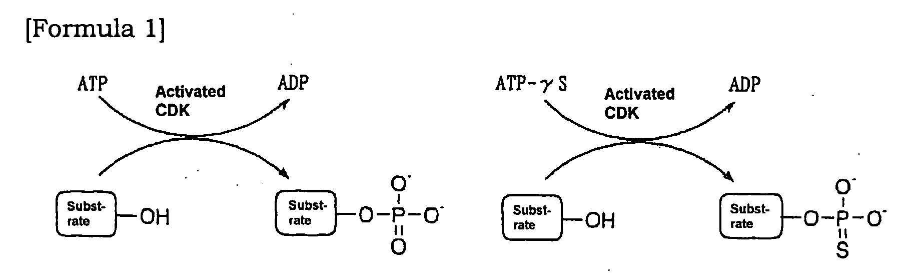

As shown above, the activated CDK which is formed by the binding of CDK with cyclin usually act to introduce an ATP-derived monophosphate group into a serine or threonine residue of the substrate protein. When ATP-γS is used instead of ATP, a monothiophosphate group instead of a monophosphate group is introduced into a serine or threonine residue of the substrate protein. Then, a sulfur atom of the monothiophosphate group introduced into the substrate protein can be labeled with a labeling fluorescent substance or a labeling enzyme and the label can be detected, thereby enabling measurement of CDK activity.

Therefore, the substrate protein may be the one having a serine or threonine residue. In a case where the activities of CDK1 or CDK2 and CDK4 or CDK6 are measured, histone H 1 and Rb (Retinoblastoma protein) are preferably used, respectively.
In a case where the substrate protein originally containing a cysteine residue in its molecule, such as Rb, is used, the residue is substituted with an amino acid residue not containing a thiol group, such as alanine. This is carried out to avoid measurement errors due to labeling of the thiol group of the cysteine residue originally contained in the substrate protein when the sulfur atom of the ATP-γS-derived thiophosphate group introduced into the substrate protein is labeled by the action of activated CDK with a labeling fluorescent substance or a labeling enzyme. As a method for obtaining such a substrate protein whose amino acid residue has been substituted, a conventional genetic engineering technique can be used. Particularly, a method disclosed in U.S. Patent Publication No. 2002/0164673 is preferably used.

Eluate obtained by introducing the substrate solution into the column in such a manner as described above is collected and a reaction product in the eluate is detected.
The detection of the reaction product can be carried out using a method usually used in a conventional method for analyzing enzyme reaction. For example, in a case where the reaction product is hydrogen peroxide, detection can be carried out by measuring an electrochemical change. In a case where the reaction product is p-nitrophenol, nucleotide, or oligopeptide, detection can be carried out by measuring a change in UV absorption, fluorescence or refractive index.
In a case where the enzyme to be measured is CDK, the reaction product can be detected using a method disclosed in U.S. Patent Publication No. 2002/0164673.

In each of the above steps, feeding of a solution or suspension into the column can be manually carried out using, for example, a syringe, but is preferably carried out using a pump at a constant flow rate.

According to the method of measuring an enzyme activity of the present invention, it is possible to measure the activity of the enzyme as long as the antibody directed against the enzyme is available. Further, even when the concentration of the enzyme in the sample is low, the antibody can capture the enzyme, and therefore the enzyme reaction can be carried out in a very small space with good reaction efficiency. Furthermore, enzyme reaction conventionally requires many steps, but the present invention simplifies it by using the technique of feeding a solution into the column. The capacity of the column to be used can be made small, and therefore the temperature distribution in the column is small and enzyme reaction can be carried out under uniform conditions.

### EXAMPLES

The present invention will be described in more detail with reference to the following Examples. However, the present invention is not limited to these Examples.

### Example 1: Measurement of activity of CDK1 and CDK2 using column

### (1) Preparation of sample

In ice bath, cells of chronic myeloid leukemia-derived cell line K-562 were lysed in a lysis solution (0.1 w/v% NP-40 (surfactant, Nonidet P-40), 50 mM Tris-HCl, pH 7.4, 5 mM EDTA, 50 mM sodium fluoride, 1 mM sodium ortho-vanadate, 100 µg/ ml protease inhibitor cocktail (manufactured by SIGMA)) in a proportion of 1 × 10⁷ cells/5 ml, by repeating suction and ejection 10 times with a 5 ml syringe provided with a 23G needle. Then, insolubles were removed by centrifugation at 15,000 rpm for 5 minutes at 4°C. In this way, the sample was prepared. The total amount of protein contained in the supernatant was measured with a DC protein kit (manufactured by Bio-Rad) using bovine IgG as a standard.

### (2) Preparation of column

20 µl of Protein A Sepharose FF (sepharose to which protein A has been bound as the antibody-binding substance) (Amersham) as the support was added to 500 µl of a buffer 1 (0.1 % NP-40, 50 mM Tris-HCl, pH 7.4), and the support was packed into a column (volumetric capacity: 20.05 mm³) having an inner diameter of 1.13 mm and a length of 20 mm with a pump at a flow rate of 100 µl/min. At this time, in this column, only the contacting part 5 of the base part 3 shown in Fig. 1 was equipped with a membrane ("Durapore" manufactured by Millipore Corporation). After the completion of packing, the same membrane was set to the contacting part 4 of the reservoir 1. The total length of the column when the reservoir 1 and the base part 3 were attached to the column body 2 was 31.5 mm.

### (3) Enzyme reaction

100 µl of a polyclonal anti-CDK1 or CDK2 antibody solution obtained by diluting a polyclonal anti-CDK1 or -CDK2 antibody (manufactured by Santa Cruz Biotechnology) with the buffer 1 so that the solution contained 2 µg of protein was fed into the column at a flow rate of 50 µl/min to immobilize the antibody onto the support. The time required for immobilizing the antibody onto the support was about 6 minutes.
100 µl of the sample containing 50 µg of protein prepared in (1) was fed into the column at a flow rate of 50 µl/min to allow CDK in the sample to be captured. The time required for carrying out this step was about 6 minutes.
Then, 400 µl of a washing solution (100 mM Tris-HCl, 100 mM NaCl, pH 7.4) was fed into the column at a flow rate of 50 µl/min to wash out the column. The time required for washing was about 13 minutes.
100 µl of a substrate mixture (histone H1 10 µg, 5mM ATP-γS, 20 mM MgCl₂, 0.1 % Triton X-100, 50 mM Tris-HCl, pH 7.4) was fed into the column at a flow rate of 10 µl/min, and the eluate was collected. The time required for carrying out this step was about 8 minutes.

### (4) Detection of reaction product

To 36 µl of the eluate, 30 µl of a binding buffer containing 150 mM Tris-HCl, pH 9.2 and 5 mM EDTA was added. Then, 20 µl of a 50 mM PEO-iodoacetyl biotin (manufactured by Pierce) solution (in a 50 mM phosphate buffer, pH 6.0) was added thereto, and the resulting mixture was incubated in a dark place at room temperature for 90 minutes. Then, an equivalent amount (86 µl) of an SDS-sample loading buffer (0.125 M Tris-HCl, pH 6.8, 4 % SDS, 10 % β-mercaptoethanol, 25 % glycerol, bromophenol blue) was added thereto and the mixture was treated at 10°C for 5 minutes.

The thus obtained resultant sample was subjected to SDS-PAGE (20 µl/lane) (pre-cast gel, 4 to 20 % gradient, 10 mm × 10 mm, Daiichi Pure Chemicals). The instructions from Daiichi Pure Chemicals were followed as the conditions for SDS-PAGE (60 mA, 40 min). After SDS-PAGE, the protein developed in the gel was electrically transferred to a PVDF membrane (10V, 30 min, Western Blotting technique). The obtained membrane was blocked with 4 w/v% BSA for 30 minutes and was washed with TBS-T for 5 minutes. Subsequently, the membrane was reacted in a solution of avidin FITC (manufactured by Pierce) (diluted 1000-fold with TBS-T) at 37°C for 30 minutes. After the completion of reaction, the membrane was washed twice with TBS-T and once with water, and was then dried. Bands of fluorescence were visualized by Molecular Imager (manufactured by Bio-Rad) to measure the intensities of the bands in RFU (relative fluorescence unit).

### Comparative Example 1: Measurement of activity of CDK1 and CDK2 using immunoprecipitation

### (1) Preparation of sample

A sample was prepared in the same manner as (1) in Example 1.

### (2) Immunoprecipitation reaction

To 100 µl of the sample containing 50 µg of protein prepared in (1), 2 µg of a polyclonal anti-CDK1 or CDK2 antibody (manufactured by Santa-Cruz Biotech) and 20 µl of Protein A Sepharose FF (manufactured by Amersham) were added to carry out immunoprecipitation at 4°C for 60 minutes.
Subsequently, the sepharose beads were washed twice with the buffer 1, once with the washing solution, and once with a buffer 2 (100 mM Tris-HCl, pH 7.4) (total 4 times). The time required for washing was 10 minutes.

### (3) Enzyme reaction

50 µl of the substrate mixture was added to carry out the enzyme reaction with shaking at 37°C for 10 minutes.

### (4) Detection of reaction product

The enzyme activity was measured in the same manner as (4) in Example 1.

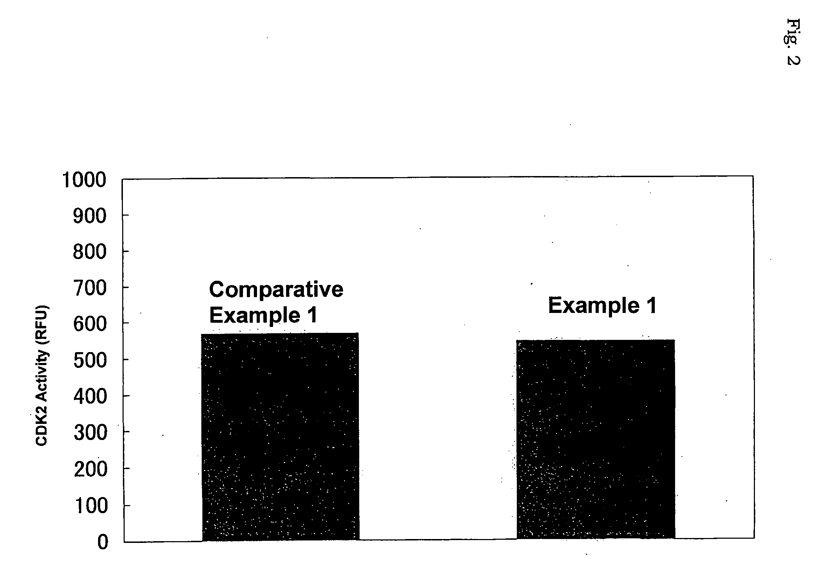

Fig. 2 shows a graph for comparison between the activities of CDK2 measured by the methods of Example 1 and Comparative Example 1. As is obvious from Fig. 2, the enzyme activity of CDK2 detected by the method of measuring an enzyme activity of the present invention using a column is substantially the same as that detected by the enzyme activity measuring method using immunoprecipitation. Further, as shown in the following Table 1, the method of measuring an enzyme activity using a column can reduce the time required for measuring the enzyme activity by more than half as compared to the enzyme activity measuring method using immunoprecipitation.

**[Table 1]**

| | Example 1 | Comparative Example 1 |
|---|---|---|
| Capture of enzyme/Immunoprecipitation | 12 minutes | 60 minutes |
| Washing | 13 minutes | 10 minutes |
| Enzyme reaction | 8 minutes | 10 minutes |
| Total | 33 minutes | 80 minutes |

### Example 2 and Comparative Example 2

The sample prepared in (1) of Example 1 was diluted so that the protein content thereof was 10, 20, or 50 µg, and the activity of CDK1 or CDK2 was measured in the same manner as in Example 1 (Example 2). Further, the same sample was diluted so that the protein content thereof was 10, 20, 50, or 100 µg, and the activity of CDK1 or CDK2 was measured in the same manner as in Comparative Example 1 (Comparative Example 2).

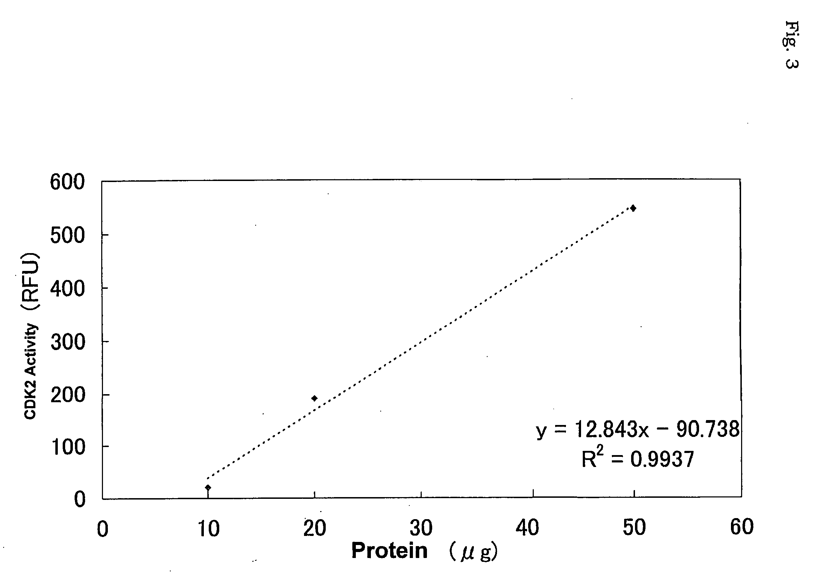

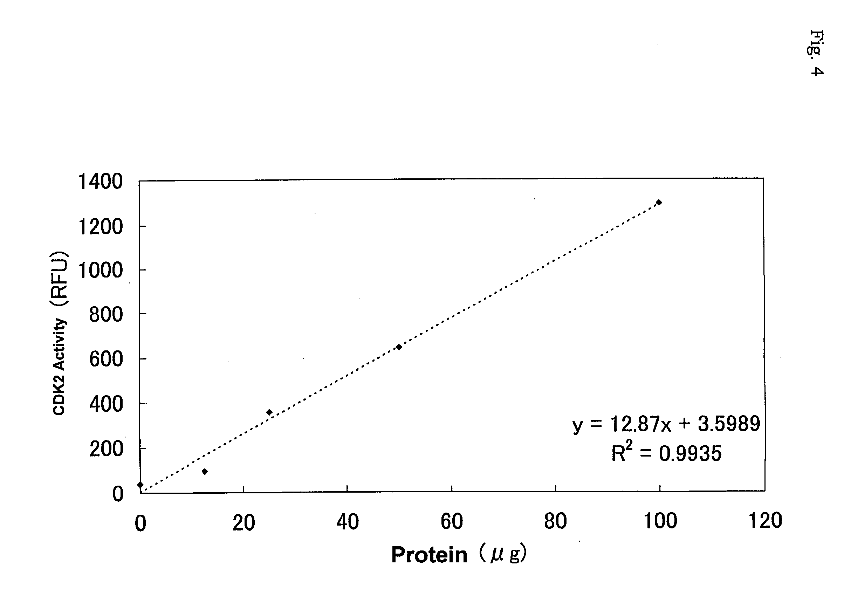

Figs. 3 and 4 show the graphs of the CDK2 activity relative to the amount of protein measured in Example 2 and Comparative Example 2.
As can be seen from Figs. 3 and 4, similar to a conventional method of measuring an enzyme activity using immunoprecipitation, the method of measuring an enzyme activity using a column is quantitative.

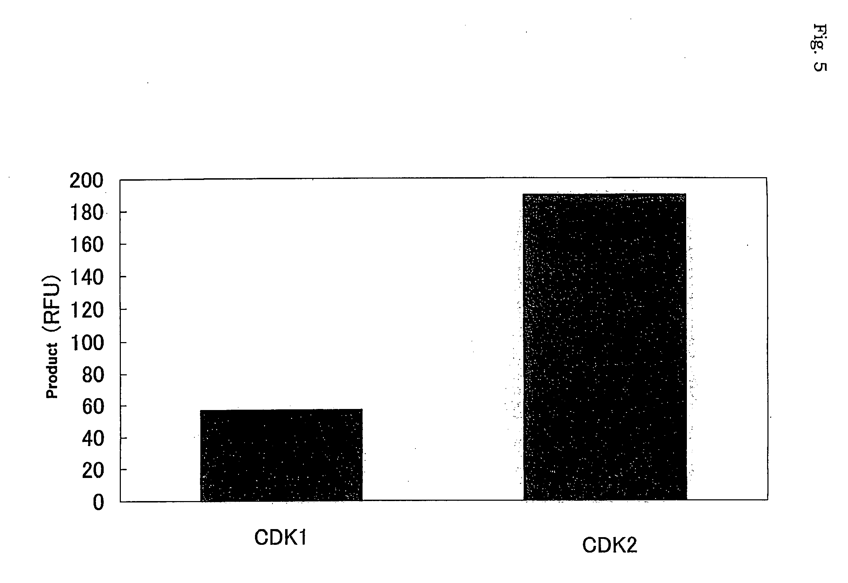

Fig. 5 shows the activity of CDK1 and CDK2 measured for the sample containing 20 µg of protein in the same method as described in Example 1.
In a case where the same method as used in Comparative Example 1 was used to measure the enzyme activity for the sample containing 20 µg of protein, CDK1 activity could not be measured because it was less than a detection limit. However, as can be seen from Fig. 5, it was possible to measure according to the method of measuring an enzyme activity using a column, thus the present method can lower the lower detection limit of CDK1 activity.

### Example 3: Measurement of activity of CDK1 and CDK2 using column

The activity of CDK1 or CDK2 was measured in the same manner as in Example 1 except that a column was prepared by mixing Protein A Sepharose FF (manufactured by Amersham) as the support and a solution of a polyclonal anti-CDK1 or CDK2 antibody (manufactured by Santa-Cruz Biotechnology) in a tube in advance, incubating the mixture to prepare the packing material, and packing the packing material in a column.

According to this Example, the time required for measuring the enzyme activity was able to be reduced as in the case of Example 1. The CDK2 activity measured in Example 3 was substantially the same as that shown in Fig. 2.

This application is based on Japanese Patent Application No. 2004-163367 (filed on June 1, 2004) whose priority is claimed, the disclosure of which including claims, specification, drawings, and abstract is incorporated herein by reference in its entirety.

### INDUSTRIAL APPLICABILITY

According to the method of measuring an enzyme activity of the present invention, it is possible to measure the enzyme activity rapidly and efficiently.
By measuring CDK activity using the method of the present invention, it is possible to diagnose cancerous diseases such as gastric cancer, colon cancer, breast cancer, lung cancer, esophageal cancer, prostate cancer, hepatic cancer, kidney cancer, bladder cancer, skin cancer, uterine cancer, cerebral tumor, osteosarcoma, and myeloma.

## Claims

1. A method of measuring an enzyme activity, which comprises the steps of:
(1) introducing a sample containing an enzyme to be measured into a column packed with a packing material comprising a water-insoluble support and an antibody which is immobilized onto the support and which recognizes the enzyme to be measured , to thereby capture the enzyme with the antibody;
(2) introducing a substrate for the enzyme into the column to react the enzyme captured by the antibody with the substrate; and
(3) detecting a product obtained by the reaction.

2. A method of measuring an enzyme activity, which comprises the steps of:
(1) introducing an antibody which recognizes an enzyme to be measured into a column packed with a water-insoluble support, to thereby immobilize the antibody onto the support;
(2) introducing a sample containing the enzyme to be measured into the column, to thereby capture the enzyme with the antibody;
(3) introducing a substrate for the enzyme into the column to react the enzyme captured by the antibody with the substrate; and
(4) detecting a product obtained by the reaction.

3. The method of measuring an enzyme activity according to claim 1 or 2, wherein the support is a particle.

4. The method of measuring an enzyme activity according to any one of claims 1 to 3, wherein the enzyme to be measured is a phosphoenzyme.

5. The method of measuring an enzyme activity according to claim 4, wherein the phosphoenzyme is a cyclin-dependent kinase.

6. A column for measuring an enzyme activity packed with a packing material comprising a water-insoluble support and an antibody which is immobilized on the support and which recognizes a phosphoenzyme.

7. The column for measuring an enzyme activity according to claim 6, wherein the phosphoenzyme is a cyclin-dependent kinase.

8. The column for measuring an enzyme activity according to claim 6 or 7, which has a diameter of 0.3 to 30 mm and a total length of 5 to 100 mm.
